# FASCICULE DE BREVET EUROPEEN

(11) **EP 0 904 086 B1**
(45) Date de publication et mention de la délivrance du brevet: **08.12.2004**
(21) Numéro de dépôt: 97928318.1
(22) Date de dépôt: 10.06.1997
(51) Int. Cl.: A61K 31/565, A61K 31/567, A61K 31/575, A61K 9/70

(54) **SYSTEMES TRANSDERMIQUES RENFERMANT DEUX PRINCIPES ACTIFS DANS DES COMPARTIMENTS SEPARES, LEUR PROCEDE DE PREPARATION ET LEUR APPLICATION COMME MEDICAMENT**
TRANSDERMALE SYSTEME ENTHALTEND ZWEI WIRKSTOFFE IN GETRENNTEN ABTEILUNGEN, IHRE HerstellungsVERFAHREN UND VERWENDUNG ALS ARZNEIMITTEL
TRANSDERMAL SYSTEMS CONTAINING TWO ACTIVE PRINCIPLES IN SEPARATE COMPARTMENTS, THEIR METHOD OF PREPARATION AND APPLICATION AS MEDICINE

(30) Priorité: 11.06.1996 FR 9607209
(43) Date de publication de la demande: 31.03.1999
(73) Titulaire: Aventis Pharma S.A., 92160 Antony (FR)
(72) Inventeur: DUBOIS, Jean-Luc, F-75019 Paris (FR)
(86) Numéro de dépôt international: PCT/FR1997/001024
(87) Numéro de publication internationale: WO 1997/047305

(56) Documents cités:
- EP-A- 0 007 823
- EP-A- 0 288 734
- WO-A-94/04157
- WO-A-94/06383

## Description

La présente invention concerne un nouveau dispositif destiné à l'administration par voie transdermique d'un principe actif associé à un autre principe actif et leur procédé de préparation.
Les demandes WO 94 04157, EP 0288 734 et WO94 06383 décrivent des systèmes d'administration transdermiques d'au moins 2 principes actifs lesdits principes actifs étant contenus dans des compartiments individualisés. C'est l'ensemble du patch qui est appliqué à la peau. Selon EP 288 734, une partie du réservoir peut le cas échéant être retiré en cours de traitement afin d'ajuster les doses et les types de principes actifs. Aucun des dispositifs décrits dans ces documents ne concerne un système transdermique qui se sépare en deux partie indépendantes devant être appliquée sur la peau, une fois le film protecteur pelable retiré.

La demanderesse a été amenée à étudier une nouvelle forme galénique :
- qui permette de réunir dans une même entité un principe actif (I) et un principe actif (II) devant être administrés de manière simultanée, séparée et étalée dans le temps pour prévenir ou traiter une maladie nécessitant une bithérapie,
- qui règle les problèmes de différence de stabilité des principes actifs au sein des polymères employés pour les couches chargées,
- qui permette d'administrer chaque principe actif dans les conditions optimum pour obtenir un flux transcutané pharmaceutiquement acceptable et évite toute interaction d'un composé avec la matrice de l'autre composé,
- qui réponde aux prescriptions requises en matière de doses et de jour d'administration de l'un et l'autre principe actif (prédosage), tout en évitant l'achat, et la manipulation de deux patchs individuels.
Dans le cadre d'une association estro-progestative, ce dernier point est particulièrement important pour le traitement hormonal substitutif de la ménopause et en particulier la prévention ou le traitement de l'ostéoporose ainsi que pour un traitement contraceptif.

A la connaissance de la demanderesse, aucune forme galénique permettant de délivrer deux principes actifs de manière simultanée, séparée et étalée dans le temps ne se présente sous la forme telle que celle proposée dans cette demande de brevet.
Selon l'état de la technique, les principes actifs sont :
- soit dans deux patchs indépendants,
- soit mélangés dans une même matrice,
- soit dans des matrices superposées,
- soit dans des matrices adjacentes sur le même film de protection.

Lorsque les principes actifs sont dans deux patchs indépendants, la prescription nécessitant la pose simultanée de ces deux patchs peut ne pas être scrupuleusement suivie, alors que si le patient trouve ces deux patchs dans une même entité, il les posera simultanément : tout risque d'oubli est ainsi écarté. Ceci facilite par ailleurs le changement simultané des deux patchs lors d'un traitement de longue durée nécessitant le renouvellement de patchs. Ceci évite ainsi tout risque de surdosage ou de sous-dosage de l'un des principes actifs par rapport à l'autre.

Lorsque les composés sont mélangés dans une même matrice, des problèmes de stabilité peuvent apparaître. De plus, il peut également y avoir des problèmes d'interaction ou de compétition qui altèrent le flux d'un des principes actifs au profit de l'autre.

Lorsque chacun des constituants est disposé dans des matrices superposées, les problèmes soulevés plus haut peuvent également apparaître.

Lorsque les matrices sont adjacentes, ils peut y avoir des problèmes de migration ou de diffusion d'une matrice à l'autre qui risquent également d'altérer le flux ou/et la stabilité d'un des principes actifs au profit de l'autre.

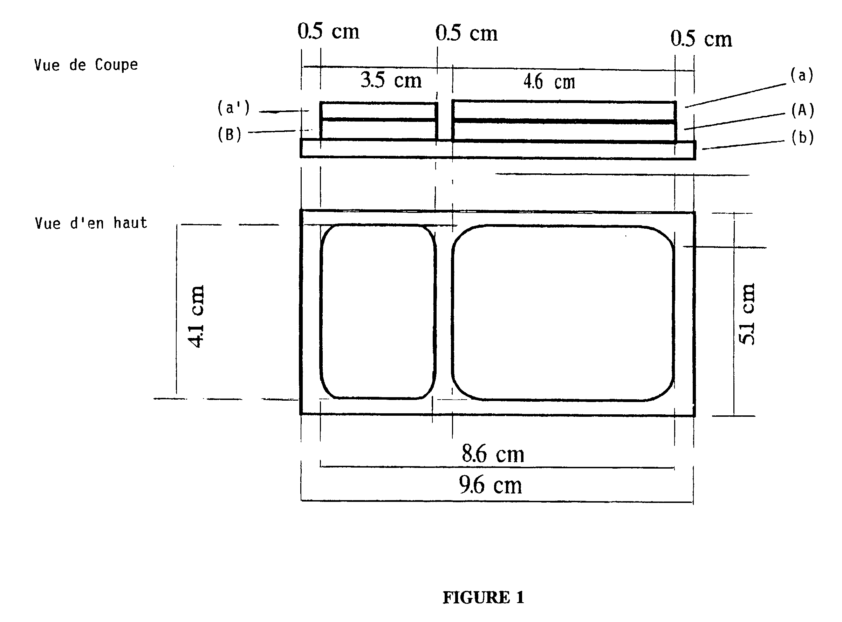

L'invention a ainsi pour objet un dispositif (cf. figure 1) destiné à l'administration transdermique d'un principe actif (I) et d'un principe actif (II), constitué de deux compartiments (A) et (B),
- le compartiment (A) renfermant une matrice polymère adhésive chargée en principe actif (I), à laquelle on adjoint éventuellement un ou plusieurs additifs,
- et le compartiment (B) renfermant une matrice polymère adhésive chargée en principe actif (II),à laquelle on adjoint éventuellement un ou plusieurs additifs, chacune de ces matrices étant respectivement recouvertes d'un film de protection (a) et (a') identique ou différent, caractérisé en ce que le compartiment (A) est séparé du compartiment (B) par un espace vide de 1 à 10 mm et caractérisé en ce que les compartiments (A) et (B) sont supportés par le même film protecteur pelable (b).

A chaque matrice polymère renfermant le principe actif peut être adjoint éventuellement un additif hydrophile et/ou un promoteur d'absorption et/ou un plastifiant et/ou tout autre additif connu de l'homme du métier permettant d'améliorer les critères de flux, d'adhésion et de stabilité des systèmes transdermiques.

Selon l'invention le compartiment (A) a une surface comprise entre 5 et 50 cm² et le compartiment (B) a une surface comprise entre 5 cm² et 50 cm².

La surface de chaque matrice (A) et (B) pourra être différente dans le but de faciliter la distinction entre les deux patchs, ou pour des raisons de doses requises.

Les dispositifs tels que décrits précédemment peuvent être de forme quelconque. Chaque compartiment (A) ou (B) peut également être de forme quelconque notamment ronde, ovale, rectangulaire ou carrée.

Le film protecteur pelable (b) est caractérisé en ce qu'il supporte les deux compartiments indépendants (A) et (B) renfermant respectivement un principe actif (I) et un principe actif (II).

Une fois ce film protecteur pelable retiré, on obtient deux patchs indépendants qu'il s'agira d'appliquer sur la peau ou une muqueuse de façon à avoir une administration simultanée, séparée et étalée dans le temps du principe actif (I) et du principe actif (II).

Selon l'invention, on peut également prévoir un ou plusieurs moyens de fixation entre les deux compartiments (A) et (B) afin d'obtenir les deux patchs liés, une fois le film protecteur pelable (b) retiré.

Le "bipatch" objet de l'invention permet ainsi d'administrer toutes les associations médicamenteuses connues de l'homme du métier dans un but de bithérapie.

L'invention s'étend également aux "bipatchs" permettant d'administrer non seulement, des associations présentant un effet de synergie ou de compensation dans un seul but thérapeutique, mais également des juxtapositions de principes actifs, chacun ayant un rôle indépendant du point de vue thérapeutique.

Ce "bipatch" objet de l'invention résout ainsi de manière particulièrement simple les différents problèmes rencontrés dans les patchs mixtes de l'art antérieur, et présente une réponse appropriée aux objectifs décrits précédemment. Il présente en outre les avantages suivants : en appliquant sur le même film protecteur pelable deux matrices indépendantes, on peut aisément :
- optimiser de manière indépendante les formulations renfermant les principes actifs (choix du polymère support, choix d'un promoteur d'absorption, choix du polymère hydrophile, choix d'un plastifiant, optimisation du grammage), en fonction des critères d'adhésion et de flux désirés,
- optimiser de manière indépendante les concentrations des principes actifs en fonction de critères de stabilité, de flux transcutanés désirés ainsi que des doses prescrites,
- obtenir des compartiments ayant des tailles identiques ou différentes, ceci par un procédé de fabrication simple.

### Film protecteur pelable (b)

Les films protecteurs pelables utilisés sont des films destinés à préserver la face adhésive à coller sur la peau du système transdermique après fabrication et durant le stockage.

Parmi les films protecteurs pelables connus de l'homme du métier, on utilisera de préférence un film polyester de type Scotchpak® 1022 (société 3M Health Care Limited), dont une des faces est traitée par des fluorocarbones ou un film polyester transparent Silox® B5Y/0 (société Akrosil™) dont une des faces est traitée anti-adhérente par des silicones Bio-release de Dow-Corning.

### Matrice polymères adhésives

Les matrices polymères adhésives renfermant un principe actif sont choisies parmi un certain nombre de polymères du commerce et/ou connus de l'homme du métier. Il s'agit notamment des polymères ou copolymères constitués par un réseau de chaînes polyisobutylène ou polyacrylique, les copolymères d'éthylène et d'acétate de vinyle (EVA) ou encore les polymères silicone. Le cas échéant on peut ajouter à ces polymères tout additif connu de l'homme du métier dans le domaine des systèmes transdermiques.

Cette matrice peut être mono ou multicouches. Les compartiments A et B peuvent également renfermer un système à réservoir.

Parmi les réseaux de chaînes polyisobutylènes utilisés on peut citer ceux qui sont commercialisés sous la marque Vistanex® (société EXXON) ou Oppanol® (société BASF).

Parmi les polymères acryliques on peut citer :
- les polymères acryliques Gelva® 737 et 788 constitués par un mélange d'acrylate de 2-éthylhexyle et d'acétate de vinyle,
- les polymères acryliques Acronal® et notamment Acronal® V205 et DS 3405,
- les solutions acryliques autoréticulables Durotak® et notamment Durotak® 126-1753, 280-2516, 380-1054,
- les copolymères d'esters acryliques et méthacryliques Eudragit® et notamment Eudragit® RL100 et S100,
- la dispersion aqueuse de méthacrylates neutres Eudragit® NE 30D.

Parmi les polymères silicone on peut citer ceux au pouvoir adhésif instantané fort (BIO PSA® 7-4301), et ceux au pouvoir adhésif instantané moyen (BIO PSA® 7-3045, 7-4201 ou 7-4202). (Société Dow Corning Health Care Centre Europe).

Le pouvoir adhésif est caractérisé par la force de pelage et la force d'adhésion : ce pouvoir adhésif augmente lorsque l'on passe du grade "low tack" à "medium tack" puis "high tack". Le paramètre permettant de moduler cette caractéristique physique est le ratio polymère/résine de silicone.

Par pouvoir adhésif moyen, on entend un rapport 40/60. Par pouvoir adhésif fort, on entend un rapport 45/55.

### Promoteurs d'absorption

Les promoteurs d'absorption éventuellement utilisés sont choisis parmi un certain nombre de promoteurs du commerce et/ou connus de l'homme du métier, notamment les éthers monoalkyliques du diéthylèneglycol, les glycérides polyglycolysés saturés constitués de glycérides et d'esters de polyéthylèneglycol d'acides gras renfermant de 6 à 14 atomes de carbone, les monoalkylates renfermant de 8 à 12 atomes de carbone, le propane-diol 1,2 et l'éthanol, ou un mélange de ces constituants.

### Additifs hydrophiles

Les additifs hydrophiles éventuellement utilisés sont choisis parmi un certain nombre de polymères du commerce et/ou connus de l'homme du métier, notamment les gommes guar, la gomme xanthane et la polyvinylpyrrolidone.

### Plastifiants

Les plastifiants éventuellement utilisés ont pour fonction d'améliorer le travail d'adhésion instantané. Il s'agit notamment de l'huile de silicone ou le cétiol S (dioctyl cyclohexane) ou le glycérol triacétate.

### Espace vide

L'espace vide permettant de séparer les deux compartiments (A) et (B) peut être compris entre 1 et 10 mm. Il est de préférence compris entre 2 et 4 mm.

### Films de protection (a) et (a')

Les films de protection utilisés sont des supports sur lesquels sont enduites les différentes couches constituant le patch pour obtenir un système indissociable. On utilise de préférence un des produits de la gamme Scotchpak® de chez 3M, et plus particulièrement le Scotchpak® 1109 qui est un film qui a la couleur de la peau, occlusif et souple, ou le Scotchpak® 1006, ou de la gamme Hostaphan® de chez Hoechst et plus particulièrement la gamme RN (RN23, RN50 ou RN75). Bien entendu les matrices peuvent être recouvertes par des films protecteurs identiques ou différents.

L'invention a plus particulièrement pour objet un dispositif destiné à l'administration transdermique tel que décrit précédemment, caractérisé en ce que le compartiment (A) renferme un composé progestomimétique et le compartiment (B) renferme un composé estrogène.

L'invention a plus spécialement pour objet les dispositifs tels que décrits précédemment caractérisés en ce que le progestomimétique est choisi parmi les composés suivants :
noréthindrone (17α)-17-hydroxy-19-norpregn-4-èn-20-yne-3-one, norgestimate (17α)-17-(acétyloxy)-13-éthyl-18,19-dinorpregn-4-èn-20-yn-3-one-3-oxyme, norgestérone (17α)-17-hydroxy-19-norpregna-5(10),20-dièn-3-one, Trimégestone 17alpha-méthyl-17béta-(2-hydroxy-1-oxo-propyl)-estra-4,9-dièn-3-one (21S), promégestone (17β)-17-méthyl-17-(1-oxopropyl)estra-4,9-dièn-3-one, Levonorgestrel Forme (-) du 13-éthyl-17-hydroxy-18,19-dinorpregn-4-èn-20-yn-3-one (norgestrel), ST 1435 16-méthylène-17α-acétoxy-19-nor-4-pregnène-3,20-dione, Médroxyprogestérone (6α)-17-hydroxy-6-méthylpregn-4-ène-3,20-dione, Gestodène (17α)-13-éthyl-17-hydroxy-18,19-dinorpregna-4,15-dièn-20-yn-3-one, Diènogest 17-hydroxy-3-oxo-19-nor-17α-pregna-4,9-diène-21-nitrile, Désogestrel (17α)-13-éthyl-11-méthylène-18,19-dinorpregn-4-èn-20-yn-17-ol, Kétodésogestrel (17α)-13-éthyl-11-méthylène-18,19-dinorpregn-4-èn-20-yn-3-one-17-ol, Acétate de noréthistérone (17α)-17-acétoxy-19-norpregn-4-èn-20-yn-3-one, Démégestone 17-méthyl-19-norpregna-4,9-diène-3,20-dione et les combinaisons de ces composés.

L'invention a tout spécialement pour objet les dispositifs tels que décrits précédemment caractérisés en ce que le composé progestomimétique est la Trimégestone.

La Trimégestone (le 17alpha-méthyl-17béta-(2-hydroxy-1-oxo-propyl)-estra-4,9-dièn-3-one (21S)) est un puissant progestomimétique décrit dans le brevet Européen EP-0007823.

L'invention a plus spécialement pour objet les dispositifs tels que décrits précédemment caractérisés en ce que le composé estrogène est choisi parmi les composés suivants : le 17-béta-estradiol, l'éthynyl estradiol, l'oestrone et l'estrogène "d'origine équine" tel que le Premarin® et les combinaisons de ces composés.

L'invention a tout spécialement pour objet les dispositifs tels que décrits précédemment caractérisés en ce que le composé estrogène est le 17β-estradiol.

Lorsque le composé estrogène est l'estradiol, la matrice chargée sera de préférence une matrice monocouche constituée d'un copolymère d'acrylate de 2-éthylhexyle et d'acétate de vinyle, à laquelle on adjoint éventuellement un polymère hydrophile. Il s'agira alors tout particulièrement du copolymère Gelva® 737 renfermant 72 % de 2-éthylhexylacrylate et 28 % de vinylacétate.

Le polymère hydrophile sera de préférence la polyvinylpyrrolidone. Il s'agira tout particulièrement du Kollidon® 30 ou 90F.

La quantité d'estradiol incorporée dans une matrice polymère telle que définie précédemment est comprise entre 1 % p/p et 10 % p/p. Ce pourcentage correspondant au poids sec de la masse enduite après évaporation du solvant.

Lorsque le composé progestomimétique est la Trimegestone, la matrice chargée sera de préférence,
- soit une matrice monocouche constituée d'un polymère silicone à laquelle on adjoint éventuellement un plastifiant tel que l'huile de silicone,
- soit une matrice bicouche,
   a) la première couche, constituée d'un polymère silicone chargé en Trimegestone, et
   b) la deuxième couche, couche d'adhésion à la peau, constituée d'un polymère silicone.

Dans tous les cas, les matrices sont de préférence constituées d'un réseau de chaîne polydiméthylsiloxane ayant un pouvoir adhésif fort tel que le BIO PSA® 7-4301.

La quantité de Trimegestone incorporée dans une matrice polymère telle que définie précédemment est comprise entre 1 % p/p et 10 % p/p. Ce pourcentage correspondant au poids sec de la masse enduite après évaporation du solvant.

La Trimegestone (le 17alpha-méthyl-17béta-(2-hydroxy 1-oxo-propyl)-estra-4,9-dièn-3-one (21S)) n'étant pas stable dans la matrice employée pour l'estradiol, le dispositif objet de l'invention est particulièrement approprié pour l'admisistration de la Trimegestone associée à l'estradiol.

L'invention a tout particulièrement pour objet les dispositifs tels que décrits précédemment présentant les caractéristiques suivantes :
**BIPATCH 1** constitué
   - d'un film protecteur pelable (b) supportant les deux compartiments (A) et (B), séparés l'un de l'autre par un espace vide de 1 à 10 mm,
   - le compartiment (A) renfermant une matrice monocouche, recouverte d'un film de protection (a), et constituée de polymère silicone chargé en Trimegestone, et éventuellement en plastifiant,
   - et le compartiment (B) renfermant une matrice monocouche, recouverte d'un film de protection (a'), et constituée d'un copolymère d'acrylate de 2-éthylhexyle et d'acétate de vinyle, chargé en estradiol, et éventuellement en polymère hydrophile.
**BIPATCH 2** constitué
   - d'un film protecteur pelable (b) supportant les deux compartiments (A) et (B), séparés l'un de l'autre par un espace vide de 1 à 10 mm,
   - le compartiment (A) renfermant une matrice bicouche recouverte d'un film de protection (a),
      a) la première couche, étant constituée d'un polymère silicone ayant un pouvoir adhésif fort chargé en Trimegestone,
      b) la deuxième couche, couche d'adhésion à la peau, étant également constituée d'un polymère silicone,
   - et le compartiment (B) renfermant une matrice monocouche, recouverte d'un film de protection (a'), et constituée d'un copolymère d'acrylate de 2-éthylhexyle et d'acétate de vinyle, chargé en estradiol et éventuellement en polymère hydrophile.

L'invention a tout particulièrement pour objet les dispositifs tels que décrits précédemment renfermant :
**BIPATCH 1a** constitué
   - d'un film protecteur pelable (b) supportant les deux compartiments (A) et (B), séparés l'un de l'autre par un espace vide de 1 à 10 mm,
   - le compartiment (A) renfermant une matrice monocouche recouverte d'un film de protection opaque (a) et constituée de 80 à 99 % p/p de polymère silicone ayant un pouvoir adhésif fort chargé en 1 à 10 % p/p de Trimegestone et en 0 à 10 % p/p d'huile de silicone ou de Cetiol® S,
   - et le compartiment (B), renfermant une matrice monocouche recouverte d'un film de protection (a') et constituée de 60 à 99 % p/p de Gelva® 737 chargé en 1 à 10 % p/p d'estradiol et en 0 à 30 % p/p de Kollidon®.
**BIPATCH 2a** constitué
   - d'un film protecteur pelable (b) supportant les deux compartiments (A) et (B), séparés l'un de l'autre par un espace vide de 1 à 10 mm,
   - le compartiment (A) renfermant une matrice bicouche recouverte d'un film de protection (a),
      a) la première couche, étant constituée de 90 à 99 % p/p d'un polymère silicone ayant un pouvoir adhésif fort, chargé en 1 à 10 % p/p de Trimegestone,
      b) la deuxième couche, couche d'adhésion à la peau, étant également constituée d'un polymère silicone ayant un pouvoir adhésif fort,
   - et le compartiment (B) renfermant une matrice monocouche, recouverte d'un film de protection (a') et constituée de 60 à 99 % p/p de Gelva® 737 chargé en 1 à 10 % p/p d'estradiol et en 0 à 30 % p/p de Kollidon®.

Selon l'invention le flux transcutané de l'estradiol est compris entre 0,1 et 2,5 µg.cm⁻².h⁻¹ et le flux transcutané de la Trimegestone est compris entre 0,1 et 3 µg.cm⁻².h⁻¹.

L'invention a également pour objet un procédé de fabrication des dispositifs tels que décrit précédemment.

La technique de fabrication des systèmes transdermiques objet de l'invention est l'enduction. Le principe général est le suivant :
**Etape I :** pour la fabrication du patch correspondant au compartiment (A)
   1 - on enduit la couche polymère adhésive silicone chargée en principe actif (I) et éventuellement en un ou plusieurs additifs tels qu'un polymère hydrophile, un promoteur d'absorption ou un plastifiant, sur le film de protection (a),
   2 - on évapore le solvant jusqu'à obtention de l'ensemble "matrice chargée en principe actif (I)/film protecteur (a)" correspondant au compartiment (A),
   3 - on effectue une opération de colaminage de l'ensemble "matrice chargée en principe actif (I)/film protecteur (a)" sur un film protecteur pelable (b'),
   4 - on découpe un patch de 5 à 50 cm².
**Etape II :** pour la fabrication du patch correspondant au compartiment (B)
   1 - on enduit la couche polymère adhésive chargée en principe actif (II) et éventuellement en un ou plusieurs additifs tels qu'un polymère hydrophile, un promoteur d'absorption ou un plastifiant, sur le film de protection (a'),
   2 - on évapore le solvant jusqu'à obtention de l'ensemble "matrice chargée en principe actif (II)/film protecteur (a')" correspondant au compartiment (B),
   3 - on effectue une opération de colaminage de l'ensemble "matrice chargée en principe actif (II)/film protecteur (a')" sur film protecteur pelable (b"),
   4 - on découpe un patch de 5 à 50 cm².
**Etape III** : pour la formation du "bipatch"
   1 - on effectue un pelage du film protecteur pelable (b') du patch obtenu à l'étape I,
   2 - puis on transfert l'ensemble "matrice chargée en principe actif (I)/film protecteur (a)" sur un film protecteur pelable (b) ,
   3 - on effectue un pelage du film protecteur pelable (b') du patch obtenu à l'étape II,
   4 - puis on transfert l'ensemble matrice chargée en "principe actif (II)/film protecteur (a')" sur le film protecteur pelable (b) précédent, en respectant une distance de 1 à 10 mm entre les deux compartiments (A) et (B).

On obtient ainsi le "bipatch" caractérisé en ce qu'il renferme (figure 1) :
- un film protecteur pelable **(b)**,
- un compartiment **(A)** constitué d'une matrice chargée en principe actif (I) et recouverte d'un film protecteur **(a)**
- un compartiment **(B)** constitué d'une matrice chargée en principe actif (II) et recouverte d'un film protecteur **(a')**, les deux compartiments étant séparés l'un de l'autre par un espace vide de 1 à 10 mm.

En association avec un estrogène, le progestatif Trimegestone manifeste une forte activité antiestrogène au niveau de l'utérus tout en ne manifestant aucune activité antiestrogène au niveau osseux.

L'association Trimegestone/estradiol telle que décrite dans cette demande peut donc être utilisée de façon simultanée, séparée et étalée dans le temps pour le traitement hormonal substitutif de la ménopause et la prévention ou le traitement de l'ostéoporose. Elle permet la prévention des symptômes et des conséquences de la ménopause comme par exemple les bouffées de chaleur, les sueurs, l'atrophie et la sécheresse vaginale, les symptômes urinaires et à long terme la diminution de la masse osseuse avec augmentation du risque de fracture et la perte de la protection cardio-vasculaire offerte par les estrogènes.

L'association estro/progestative telle que décrite dans cette demande peut également être utilisée comme contraceptif.

L'invention a donc enfin pour objet un dispositif tel que décrit précédemment, pour l'utilisation dans un procédé de délivrance de plusieurs médicaments par application des deux matrices du dispositif à la peau ou à une muqueuse dudit patient.

### Exemples de traitements utilisant le bipatch

Les exemples de traitement ci-dessous illustrent l'invention sans toutefois la limiter. Pour chaque bipatch, la durée d'adhésion est de 4 à 7 jours.

### 1. Trimegestone associée avec l'estradiol

Dans le cadre d'un traitement hormonal substitutif de la ménopause et en particulier dans la prévention ou le traitement de l'ostéoporose.

### 1.1 Administration de la Trimegestone en séquentiel et de l'estradiol en continu :

### Traitement a

Administration de l'estradiol en continu (cycles de 28 jours sans interruption entre les cycles) à une dose de 25 à 200 µg par jour et de la Trimegestone les 14 derniers jours de chaque cycle de 28 jours, à une dose de 0,05 à 2,5 mg par jour. Le bipatch trimegestone/estradiol selon l'invention est donc utilisé les 14 derniers jours (soit 2 à 4 bipatchs).

### Traitement b

Administration de l'estradiol 28 jours par mois à une dose de 25 à 200 µg par jour et de la Trimegestone les 14 derniers jours de l'administration de l'estradiol à une dose de 0,05 à 2,5 mg par jour. Le traitement est arrêté 3 jours par mois à la fin de chaque cycle de 28 jours. Le bipatch trimegestone/estradiol selon l'invention est donc utilisé les 14 derniers jours (soit 2 à 4 bipatchs).

### Traitement c

Administration de l'estradiol 28 jours par mois à une dose de 25 à 200 µg par jour et du patch Trimegestone les 14 premiers jours de l'administration de l'estradiol à une dose de 0,05 à 2,5 mg par jour. Le traitement est administré soit sans interruption entre chaque cycle de 28 jours, soit avec interruption de 2 à 3 jours par mois à la fin de chaque cycle. Le bipatch trimegestone/estradiol selon l'invention est donc utilisé les 14 premiers jours (soit 2 à 4 bipatchs).

### Traitement d

Administration de l'estradiol 25 jours par mois à une dose de 25 à 200 µg par jour et de la Trimegestone à une dose de 0,05 à 2,5 mg par jour pendant les 11 à 14 derniers jours de l'administration de l'estradiol. Le traitement est arrêté 5 à 6 jours à la fin de chaque cycle de 25 jours.

Le bipatch trimegestone/estradiol selon l'invention est donc utilisé les 11 à 14 derniers jours (soit 2 à 4 bipatchs)

### 1.2 Administration en continu de la Trimegestone et de l'estradiol

Administration continue transdermique de l'estradiol à une dose de 25 à 200 µg par jour et de la trimegestone à une dose de 0,05 à 2,5 mg par jour. Il n'y a pas d'interruption de traitement. Le bipatch trimegestone/estradiol selon l'invention est donc utilisé durant les 28 jours (soit de 4 à 8 bipatchs)

### 2) Trimegestone associée à l'éthynylestradiol

Dans le cadre d'un usage à titre de contraceptif.

On administre par voie transdermique l'association en continu de la Trimegestone et de l'éthynylestradiol de 21 à 28 jours par cycles. Ce traitement nécessite ainsi l'application successive de 3 à 8 bipatchs Trimegestone/éthynyl estradiol.

Des exemples de bipatchs selon l'invention figurent ci-après dans la partie expérimentale.

### Exemple 1 :

### Patch renfermant la Trimegestone associée à l'estradiol BIPATCH 1b

Ce bipatch comporte les caractéristiques suivantes :
- un film protecteur pelable Scotchpak® 1022 supportant les deux compartiments (A) et (B), séparés l'un de l'autre par un intervalle de 2 à 4 mm
- le compartiment (A) renferme une matrice monocouche, recouverte d'un film de protection opaque Scotchpak® 1006 et constituée de 96 % p/p de polymère silicone ayant un pouvoir adhésif instantané fort chargée en 3 % p/p de Trimegestone et en 1 % d'huile de silicone (7-9120, 12000 cSt). Le grammage est égal à 60 g/m².
- et le compartiment (B), renferme une matrice monocouche, recouverte d'un film de protection Scotchpack® 1109 ou Hostaphan® RN23, et constituée de 73 % p/p d'une couche Gelva® 737 chargé en 2 % p/p d'estradiol et en 25 % p/p de Kollidon® 90F.
Le grammage est égal à 80 g/m².

### BIPATCH 2b

Ce bipatch comporte les caractéristiques suivantes :
- un film protecteur pelable Scotchpak® 1022 supportant les deux compartiments (A) et (B), séparés l'un de l'autre par un intervalle de 2 à 4 mm,
- le compartiment (A) renferme une matrice bicouche recouverte d'un film de protection Scotchpak® 1006,
   a) la première couche de grammage 60 g/m², chargée en 3 % p/p de Trimegestone, étant constituée de 97 % p/p d'un polymère silicone ayant un pouvoir adhésif instantané fort,
   b) la deuxième couche, couche d'adhésion à la peau, étant constituée d'un polymère silicone ayant un pouvoir adhésif instantané fort ; son grammage est de 30 g/m². Le grammage total est alors égal à 90 g/m².
- et le compartiment (B) renferme une matrice monocouche, recouverte d'un film de protection Scotchpak® 1109 ou Hostaphan® RN23 constituée de 73 % p/p d'une couche Gelva® 737 chargé en 2 % p/p d'estradiol et en 25 % p/p de Kollidon® 90F. Le grammage est alors égal à 80 g/m².

### Exemple 2 : Quantité de principe actif dans chaque compartiment

La quantité x de principe actif peut être exprimée par la formule générale suivante
soit
G, exprimé en mg/cm², exprimant le grammage total de la masse enduite (île somme des grammages de chaque couche enduite dans le cas d'un patch multicouches)
S, la surface du patch exprimée en cm²
p, le pourcentage en principe actif dans l'ensemble de la masse enduite, exprimé en % p/p,
alors, la masse enduite, notée M, se calcule par M=G . S exprimée en mg/patch.
D'où x = p.M/100 = p.G.S/100 exprimée en mg/patch

| | compartiment (A) renfermant le principe actif (I) | compartiment (B) renfermant le principe actif (II) |
|---|---|---|
| G (mg/cm²) | 6 | 8 |
| p (%) | 3 | 2 |
| S mini (cm²) | 5 | 5 |
| S maxi (cm²) | 50 | 50 |
| x mini (mg/patch) | 0,9 | 0,8 |
| x maxi (mg/patch) | 9 | 8 |

Figure n° 1 :
(B) Compartiment renfermant le principe actif (II)
(A) Compartiment renfermant le principe actif (I)
(a) Film protecteur
(a') Film protecteur
(b) Film protecteur pelable.

## Revendications

1. Dispositif destiné à l'administration transdermique d'un principe actif (I) et d'un principe actif (II), constitué de deux compartiments (A) et (B),
- le compartiment (A) renfermant une matrice polymère adhésive chargée en principe actif (I) à laquelle on adjoint éventuellement un ou plusieurs additifs,
- et le compartiment (B) renfermant une matrice polymère adhésive chargée en principe actif (II) à laquelle on adjoint éventuellement un ou plusieurs additifs, chacune de ces matrices étant rapidement recouvertes d'un film de protection (a) et (a') identique ou différent, **caractérisé en ce que** le compartiment (A) est séparé du compartiment (B) par un espace vide de 1 à 10 mm et **caractérisé en ce que** les compartiments (A) et (B) sont supportés par le même film protecteur pelable (b).

2. Dispositif destiné à l'administration transdermique selon la revendication 1, **caractérisé en ce que** le compartiment (A) renferme un composé progestomimétique et le compartiment (B) renferme un composé estrogène.

3. Dispositif selon la revendication 2, **caractérisé en ce que** le progestomimétique est choisi parmi les composés suivants :
noréthindrone (17α)-17-hydroxy-19-norpregn-4-èn-20-yne-3-one, norgestimate (17α)-17-(acétyloxy)-13-éthyl-18,19-dinorpregn-4-èn-20-yn-3-one-3-oxyme, norgestérone (17α)-17-hydroxy-19-norpregna-5(10),20-dièn-3-one, Trimégestone 17alpha-méthyl-17béta-(2-hydroxy-1-oxo-propyl)-estra-4,9-dièn-3-one (21S), promégestone (17β)-17-méthyl-17-(1-oxopropyl)estra-4,9-dièn-3-one, Levonorgestrel Forme (-) du 13-éthyl-17-hydroxy-18,19-dinorpregn-4-èn-20-yn-3-one (norgestrel), ST 1435 16-méthylène-17α-acétoxy-19-nor-4-pregnène-3,20-dione, Médroxyprogestérone (6α)-17-hydroxy-6-méthylpregn-4-ène-3,20-dione, Gestodène (17α)-13-éthyl-17-hydroxy-18,19-dinorpregna-4,15-dièn-20-yn-3-one, Diènogest 17-hydroxy-3-oxo-19-nor-17α-pregna-4,9-diène-21-nitrile, Désogestrel (17α)-13-éthyl-11-méthylène-18,19-dinorpregn-4-èn-20-yn-17-ol, Kétodésogestrel (17α)-13-éthyl-11-méthylène-18,19-dinorpregn-4-èn-20-yn-3-one-17-ol, Acétate de noréthistérone (17α)-17-acétoxy-19-norpregn-4-èn-20-yn-3-one, Démégestone 17-méthyl-19-norpregna-4,9-diène-3,20-dione et les combinaisons de ces composés.

4. Dispositif selon l'une des revendications 2 et 3, **caractérisé en ce que** le composé progestomimétique est la Trimegestone.

5. Dispositif selon la revendication 2, **caractérisé en ce que** le composé estrogène est choisi parmi les composés suivants :
le 17-béta-estradiol, l'éthynyl estradiol, l'oestrone et
l'estrogène "d'origine équine" tel que le Premarin®, et les combinaisons de ces composés.

6. Dispositif selon l'une des revendications 2 et 5, **caractérisé en ce que** le composé estrogène est le 17-béta-estradiol.

7. Dispositif selon l'une quelconque des revendications 1 à 6, dans lequel :
- le compartiment (A) renferme une matrice monocouche constituée de polymère silicone chargé en Trimegestone et éventuellement en plastifiant
- et le compartiment (B) renferme une matrice monocouche constituée d'un copolymère d'acrylate de 2-éthylhexyle et d'acétate de vinyle, chargé en estradiol et éventuellement en polymère hydrophile.

8. Dispositif selon l'une quelconque des revendications 1 à 6, dans lequel :
- le compartiment (A) renferme une matrice bicouche
a) la première couche, étant constituée d'un polymère silicone chargé en Trimegestone,
b) la deuxième couche, couche d'adhésion à la peau, étant également constituée d'un polymère silicone,
- et le compartiment (B) renferme une matrice monocouche, constituée d'un copolymère d'acrylate de 2-éthylhexyle et d'acétate de vinyle, chargé en estradiol et éventuellement en polymère hydrophile.

9. Procédé de fabrication des dispositifs selon les revendications 1 à 8, **caractérisé en ce que** :
**Etape I :** pour la fabrication du patch correspondant au compartiment (A)
1 - on enduit la couche polymère adhésive silicone chargée en principe actif (I) et éventuellement en un ou plusieurs additifs tels qu'un polymère hydrophile, un promoteur d'absorption ou un plastifiant, sur le film de protection (a),
2 - on évapore le solvant jusqu'à obtention de l'ensemble "matrice chargée en principe actif (I)/film protecteur (a)" correpondant au compartiment (A),
3 - on effectue une opération de colaminage de l'ensemble "matrice chargée en principe actif (I)/film protecteur (a)" sur un film protecteur pelable (b'),
4 - on découpe un patch de 5 à 50 cm².
**Etape II :** pour la fabrication du patch correspondant au compartiment (B)
1 - on enduit la couche polymère adhésive chargée en principe actif (II) et éventuellement en un ou plusieurs additifs tels qu'un polymère hydrophile, un promoteur d'absorption ou un plastifiant, sur le film de protection (a'),
2 - on évapore le solvant jusqu'à obtention de l'ensemble "matrice chargée en principe actif (II)/film protecteur (a')" correspondant au compartiment (B),
3 - on effectue une opération de colaminage de l'ensemble "matrice chargée en principe actif (II)/film protecteur (a)" sur film protecteur pelable (b"),
4 - on découpe un patch de 5 à 50 cm².
**Etape III :** pour la formation du "bipatch"
1 - on effectue un pelage du film protecteur pelable (b') du patch obtenu à l'étape I,
2 - puis on transfert l'ensemble "matrice chargée en principe actif (I)/film protecteur (a')" sur un film protecteur pelable (b),
3 - on effectue un pelage du film protecteur pelable (b") du patch obtenu à l'étape II,
4 - puis on transfert l'ensemble matrice chargée en "principe actif (II)/film protecteur (a')" sur le film protecteur pelable (b) précédent, en respectant une distance de 1 à 10 mm entre les deux compartiments (A) et (B).

10. Dispositif selon l'une quelconque des revendications 1 à 8, pour l'utilisation dans un procédé de délivrance de plusieurs médicaments par application des deux matrices du dispositif à la peau ou à une muqueuse dudit patient.

## Patentansprüche

1. Vorrichtung zur transdermalen Verabreichung eines aktiven Prinzips (I) und eines aktiven Prinzips (II) bestehend aus zwei Fächern (A) und (B), wobei
- das Fach (A) eine Polymermatrix mit Hafteigenschaften umschließt, die mit dem aktiven Prinzip (I) beschickt ist, dem eventuell ein oder mehrere Additive zugefügt werden,
- und das Fach (B) eine Polymermatrix mit Hafteigenschaften umschließt, die mit dem aktiven Prinzip (II) beschickt ist, der eventuell ein oder mehrere Additive zugefügt werden, wobei jede dieser Matrizen rasch mit einem Schutzfilm (a) und (a') von identischer oder verschiedener Natur wieder bedeckt werden,
**dadurch gekennzeichnet, dass** das Fach (A) von dem Fach (B) durch einen leeren Raum von 1 bis 10 mm abgetrennt ist und **dadurch gekennzeichnet, dass** die Fächer (A) und (B) von demselben abziehbaren Schutzfilm (b) gehalten werden.

2. Vorrichtung zur transdermalen Verabreichung nach Anspruch 1, **dadurch gekennzeichnet, dass** das Fach (A) ein Progestativum und das Fach (B) eine Estrogenverbindung umschließt.

3. Verfahren nach Anspruch 2, **dadurch gekennzeichnet, dass** das Progestativum unter den nachfolgenden Verbindungen ausgewählt ist:
Norethindron (17α)-17-Hydroxy-19-norpregn-4-en-20-yn-3-on,
Norgestimat (17α)-17-(Acetyloxy)-13-Ethyl-18, 19-dinorpregn-4-en-20-yn-3-on-3-oxim,
Norgesteron (17α)-17-Hydroxy-19-norpregna-5 (10), 20-dien-3-on,
Trimegestron (17α)-17-methyl-17beta-(2-hydroxy-1-oxo-propyl)-estra-4, 9-dien-3-on (21 S),
Promegestron (17β)-17-Methyl-17-(1-oxopropyl)estra-4, 9-dien-3-on,
Levonorgestrel (-)-Form des 13-Ethyl-17-hydroxy-18, 19-dinorpregn-4-en-20-yn-3-on (Norgestrel),
ST 1435 16-Methylen-17α-acetoxy-19-nor-4-pregnen-3, 20-dion,
Medroxyprogesteron (6α)-17-hydroxy-6-methylpregn-4-en-3, 20-dion,
Gestoden (17α)-13-Ethyl-17-hydroxy-18, 19-dinorpregna-4, 15-dien-20-yn-3-on,
Dienogest 17-Hydroxy-3-oxo-19-nor-17α-pregna-4, 9-dien-21-nitril,
Desogestrel (17α)-13-Ethyl-11-methylen-18, 19-dinorpregn-4-en-20-in-17-ol,
Ketodesogestrel (17α)-13-Ethyl-11-methylen-18, 19-dinorpregn-4-en-20-in-3-on-17-ol,
Noresthisteronacetat (17α)-17-Acetoxy-19-norpregen-4-en-20-in-3 -on,
Demegeston 17-Methyl-19-norpregna-4,9-dien-3, 20-dion, sowie Kombinationen der genannten Verbindungen.

4. Vorrichtung nach einem der Ansprüche 2 und 3, **dadurch gekennzeichnet, dass** das Progestativum Trimegeston ist.

5. Vorrichtung nach Anspruch 2, **dadurch gekennzeichnet, dass** die Estrogen-Verbindung unter den folgenden Verbindungen ausgewählt ist: 17β-Estradiol, Ethynyl-Estradiol, Östron und Pferde-Estrogen wie Premarin®, sowie Kombinationen der genannten Verbindungen.

6. Vorrichtung nach einem der Ansprüche 2 und 5, **dadurch gekennzeichnet, dass** die Estrogen-Verbindung 17β-Estradiol ist.

7. Vorrichtung nach einem der Ansprüche 1 bis 6, bei der:
- das Fach (A) eine einlagige Matrix aus Silikonpolymer beladen mit Trimegeston und eventuell Weichmacher umschließt
- und das Fach (B) eine einlagige Matrix aus einem Copolymer von 2-Ethylhexylacrylat und Vinylacetat beschickt mit Estradiol und eventuell einem hydrophilen Polymeren umschließt.

8. Vorrichtung nach einem der Ansprüche 1 bis 6, bei der:
- das Fach (A) eine zweilagige Matrix umfasst mit einer ersten Lage (a) aus einem Silikonpolymeren beschickt mit Trimegeston, die zweite Schicht (b), ebenfalls aus einem Silikonpolymeren, eine Haut-Haftschicht darstellt,
- und das Fach (B) eine einlagige Matrix aus einem Copolymeren von 2-Ethylhexylacrylat und Vinylacetat beschickt mit Estradiol und eventuell einem hydrophilen Polymeren umschließt.

9. Verfahren zur Herstellung der Vorrichtungen nach den Ansprüchen 1 bis 8, **dadurch gekennzeichnet, dass**:
**Stufe I:** Für die Herstellung des dem Fach (A) entsprechenden Patches
1- man streicht das Silikonpolymer mit Hafteigenschaften, das mit dem aktiven Prinzip (I) und eventuell einem oder mehreren Additiven wie einem hydrophilen Polymer, einem Haftverbesserer oder einem Weichmacher beschickt ist, auf den Schutzfilm (a),
2- man verdampft das Lösungsmittel bis zum Erhalt der Gesamtheit "Matrix mit aktivem Prinzip (I) beschickt / Schutzfilm (a)", entsprechend dem Fach (A),
3- ein gemeinsamer Walzgang der Gesamtheit "Matrix mit aktivem Prinzip (I) beladen /Schutzfilm (a)" auf einen abziehbaren Schutzfilm (b') wird durchgeführt,
4- ein Patch von 5 bis 50 cm² wird ausgeschnitten.
**Stufe II:** Für die Herstellung des Patches, der dem Fach (B) entspricht
1- man streicht das Silikonpolymer mit Hafteigenschaften, das mit dem aktiven Prinzip (II) und eventuell einem oder mehreren Additiven wie einem hydrophilen Polymer, einem Haftverbesserer oder einem Weichmacher beschickt ist, auf den Schutzfilm (a'),
2- man verdampft das Lösungsmittel bis zum Erhalt der Gesamtheit "Matrix mit aktivem Prinzip (II) beschickt / Schutzfilm (a')", entsprechend dem Fach (B),
3- ein gemeinsamer Walzgang der Gesamtheit "Matrix mit aktivem Prinzip (II) beladen /Schutzfilm (a')" auf einen abziehbaren Schutzfilm (b") wird durchgeführt,
4- ein Patch von 5 bis 50 cm² wird ausgeschnitten.
**Stufe III:** für die Herstellung des "Bipatch"
1- der abziehbare Schutzfilm (b') des in Stufe I erhaltenen Patches wird abgezogen,
2- anschließend wird die Gesamtheit "mit aktivem Prinzip (I) beschickte Matrix / Schutzfilm (a')" auf einen abziehbaren Schutzfilm (b) übertragen,
3- der abziehbare Schutzfilm (b") des in Stufe II erhaltenen Patches wird abgezogen,
4- anschließend wird die Gesamtheit "mit aktivem Prinzip (II) beladene Matrix / Schutzfilm (a')" auf den vorhergehenden Schutzfilm (b) übertragen, wobei eine Distanz von 1 bis 10 mm zwischen den beiden Fächern (A) und (B) eingehalten wird.

10. Vorrichtung nach einem der Ansprüche 1 bis 8 zur Verwendung in einem Verfahren der Verabreichung mehrerer Medikamente durch Aufbringen der beiden Matrizen der Vorrichtung auf die Haut oder eine Schleimhaut des Patienten.

## Claims

1. Device intended for the transdermal administration of an active ingredient (I) and an active ingredient (II), constituted by two compartments (A) and (B),
- compartment (A) containing an adhesive polymer matrix charged with active ingredient (I) to which one or more additives are optionally added,
- and compartment (B) containing an adhesive polymer matrix charged with active ingredient (II) to which one or more additives are optionally added, each one of these matrices being rapidly covered with a protective film (a) and (a') which are identical or different, **characterized in that** compartment (A) is separated from compartment (B) by an empty space of 1 to 100 mm and **characterized in that** compartments (A) and (B) are supported by the same peel-off protective film (b).

2. Device intended for transdermal administration according to claim 1, **characterized in that** compartment (A) contains a progestomimetic compound and compartment (B) contains an oestrogen compound.

3. Device according to claim 2, **characterized in that** the progestomimetic is chosen from the following compounds:
norethindrone (17α)-17-hydroxy-19-norpregn-4-en-20-yn-3-one, norgestimate (17α)-17-(acetyloxy)-13-ethyl-18,19-dinorpregn-4-en-20-yn-3-one-3-oxime, norgesterone (17α)-17-hydroxy-19-norpregna-5(10),20-dien-3-one, Trimegestone 17alpha-methyl-17beta-(2-hydroxy-1-oxo-propyl)-estra-4,9-dien-3-one (21S), promegestone (17β)-17-methyl-17-(1-oxypropyl)estra-4,9-dien-3-one, Levonorgestrel, a (-)form of 13-ethyl-17-hydroxy-18,19-dinorpregn-4-en-20-yn-3-one (norgestrel), ST 1435 16-methylene-17a-acetoxy-19-nor-4-pregnene-3,20-dione, Medroxyprogesterone (6α)-17-hydroxy-6-methylpregn-4-ene-3,20-dione, Gestodene (17α)-13-ethyl-17-hydroxy-18,19-dinorpregn-4,15-dien-20-yn-3-one, Dienogest 17-hydroxy-3-oxo-19-nor-17α-pregna-4,9-diene-21-nitrile, Desogestrel (17α)-13-ethyl-11-methylene-18,19-dinorpregn-4-en-20-yn-17-ol, Ketodesogestrel (17α)-13-ethyl-11-methylene-18,19-dinorpregn-4-en-20-yn-3-one-17-ol, norethisterone acetate (17α)-17-acetoxy-19-norpregn-4-en-20-yn-3-one, Demegestone 17-methyl-19-norpregna-4,9-diene-3,20-dione and the combinations of these compounds.

4. Device according to one of claims 2 or 3, **characterized in that** the progestomimetic compound is Trimegestone.

5. Device according to claim 2, **characterized in that** the oestrogen compound is chosen from the following compounds: 17-beta-estradiol, ethynyl estradiol, oestrone and oestrogen "of equine origin" such as Premarin®, and the combinations of these compounds.

6. Device according to one of claims 2 and 5, **characterized in that** the oestrogen compound is 17-beta-estradiol.

7. Device according to any one of claims 1 to 6, in which:
- compartment (A) contains a monolayer matrix constituted by silicone polymer charged with Trimegestone and optionally a plasticizer
- and compartment (B) contains a monolayer matrix constituted by a 2-ethylhexyl acrylate and vinyl acetate copolymer, charged with estradiol and optionally a hydrophilic polymer.

8. Device according to any one of claims 1 to 6, in which:
- compartment (A) contains a bilayer matrix
a) the first layer, being constituted by a silicone polymer charged with Trimegestone,
b) the second layer, the layer which adheres to the skin, also being constituted by a silicone polymer,
- and compartment (B) contains a monolayer matrix constituted by a 2-ethylhexyl acrylate and vinyl acetate copolymer, charged with estradiol and optionally a hydrophilic polymer.

9. Process for producing the devices according to claims 1 to 8, **characterized in that**:
**Stage I:** for the production of the patch corresponding to compartment (A)
1 - the silicone adhesive polymer layer charged with active ingredient (I) and optionally with one or more additives such as a hydrophilic polymer, an absorption promoter or a plasticizer, is coated onto the protective film (a),
2 - the solvent is evaporated off until the whole "matrix charged with active ingredient (I)/protective film (a)" corresponding to compartment (A) is obtained,
3 - an operation is carried out to colaminate the whole "matrix charged with active ingredient (I)/protective film (a)" onto a peel-off protective film (b'),
4 - a patch of 5 to 50 cm² is cut out.
**Stage II:** for the production of the patch corresponding to compartment (B)
1 - the adhesive polymer layer charged with active ingredient (II) and optionally with one or more additives such as a hydrophilic polymer, an absorption promoter or a plasticizer, is coated onto the protective film (a'),
2 - the solvent is evaporated off until the whole "matrix charged with active ingredient (II)/protective film (a')" corresponding to compartment (B) is obtained,
3 - an operation is carried out to colaminate the whole "matrix charged with active ingredient (II)/protective film (a)" onto a peel-off protective film (b"),
4 - a patch of 5 to 50 cm² is cut out.
**Stage III:** for the formation of the "bipatch"
1 - the peel-off protective film (b') is peeled off the patch obtained in Stage I,
2 - then the whole "matrix charged with active ingredient (I)/protective film (a')" is transferred onto a peel-off protective film (b),
3 - the peel-off protective film (b") is peeled off the patch obtained in Stage II,
4 - then the whole "matrix charged with active ingredient (II)/protective film (a')" is transferred onto the preceding peel-off protective film (b), keeping a distance of 1 to 10 mm between the two compartments (A) and (B).

10. Device according to any one of claims 1 to 8, for use in a process for delivering several medicaments by application of the two matrices of the device to the skin or to a mucous membrane of said patient.
